# EUROPEAN PATENT APPLICATION

(11) **EP 0 619 323 A1**
(43) Date of publication of application: **12.10.1994**
(21) Application number: 93400944.0
(22) Date of filing: 09.04.1993
(51) Int. Cl.: C07K 15/00, A61K 39/395, C12P 21/08

(54) **Human monoclonal antibodies and processes and materials for making such antibodies**

(71) Applicant: SCHERING-PLOUGH, F-92307 Levallois-Perret Cédex (FR)
(72) Inventor: Lebecque, Serge L. E., F-69380 Chazay D'Azergues (FR); Rousset, Françoise M.E., F-69005 Lyon (FR); Banchereau, Jacques, F-69130 Ecully (FR)
(74) Representative: Durand, Yves Armand Louis

(57) **Abstract**

An anti-allergic agent is disclosed which comprises a human monoclonal antibody which binds to an airborne allergen that provokes an allergic IgE response, an allergen-binding fragment of such human monoclonal antibody or an immune complex of such human monoclonal antibody or allergen-binding fragment with said allergen. Pharmaceutical compositions and methods employing the anti-allergic agent, cell lines for making the human monoclonal antibodies and purified and isolated DNA for making the human monoclonal antibodies or allergen-binding fragments thereof are also disclosed.

Also disclosed is a process for making multiple clones from an immortalized B-cell population, each of which clones secretes a human monoclonal antibody that binds to the desired antigen.

## Description

### BACKGROUND OF THE INVENTION

The present invention relates to anti-allergic agents which bind to airborne allergens, which agents are selected from human monoclonal antibodies (HuMAbs) or allergen-binding fragments or immune complexes of the human monoclonal antibodies or fragments with said allergen. The invention also relates to processes, cells and nucleic acids (e.g., cDNA, recombinant vectors, etc.) for making such anti-allergic agents. In addition, the invention relates to a process for making a human monoclonal antibody to a desired antigen.

Many airborne allergens cause degranulation of IgE-sensitized basophils/mast cells that results in allergic symptoms (such as rhinitis, conjunctivitis, atopic dermatitis and pollen-induced asthma) in sensitized individuals. These allergens include tree and grass (e.g., ragweed) pollens, house dust mites, cat and dog dander, etc.

For example, proteins isolated from aqueous extracts of birch pollen are responsible for most of the allergic symptoms occurring the middle, eastern and northern Europe, as well as in certain areas of North America during the early and middle parts of the spring season. Twenty-two percent of allergic patients in Central Europe suffer from birch pollinosis.

Many airborne allergens such as the two major allergens from birch pollen have been characterized at the molecular level. For example, Bet v 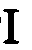 is a 17 kD glycoprotein, which has been localized on ultra-thin sections of pollen grain in the cytoplasmic matrix (Grote, M., *J Histochem Cytochem*, *39* : 1395-401 (1991). Ninety-seven per cent of patients with birch pollen allergy have IgE antibodies that recognize Bet v 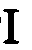 (Valenta, et al., *J Allergy Clin Immunol*, *88* : 889-94 (1991c).

The gene coding for Bet v 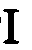 has been cloned and found to be very homologous to the pea disease resistant response gene (Breiteneder, et al., *Embo J*, *8* : 1935-8 (1989). The N-terminus of the protein is not subjected to post-translational processing.

Bet v 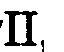 , the second major birch allergen, is recognized by the IgE antibodies of 9% of patients allergic to birch (Valenta, 1991c, *supra*). Bet v 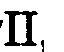 relates to profilin, an ubiquitous component of the cytoskeleton which constitutes a family of functional plant pan-allergens, that is present in trees, grasses and weeds. Approximately 20% of all patients allergic to pollen also respond to the profilins (Valenta, et al., *Science*, *253* : 557-60 (1991b). Both recombinant Bet v 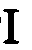 and Bet v 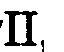 allergens expressed in *E*. *coli* are recognized by IgE from birch pollen allergic patients (Valenta, 1991c, *supra*).

The molecular characterization of Bet v 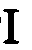 and of Bet v 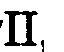 helps to explain the common observation that patients allergic to birch pollen are often sensitive to other related tree pollens. A high degree of homology at the nucleic acid level among the major allergens from birch (Bet v 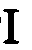 ), alder (Aln g 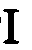 ), hazel (Cor a 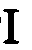 ), and hornbeam (Car b 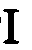 ) at the nucleic acid level has been reported (Valenta, et al., *J Allergy Clin Immunol*, *87* : 677-82 (1991a). Partial N-terminal amino acid sequence analysis of Bet v 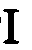 , Aln g 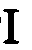 , Cor a 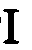 and Car b 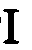 (40-45 amino acid residues) demonstrated high sequence identity-homology and putative common epitopes (Ipsen, H. and O. Hanssen, "The NH₂ Terminal Amino-Acid Sequence of The Major Allergen of Birch (*Betulaverrucosa)*,Bet v 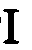 , And Alder (*Alnus Glutinosa*) Aln g 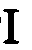 Pollens," 16th Norkiske Allergological Congress, Tromso, 1987. Accordingly, immunotherapy with birch pollen extract has been found effective in reducing IgE response as well as skin and nasal sensitivity in patients allergic to birch, hazel or hornbeam pollen (Wihl, et al., *Allergy*, *43* : 363-9 (1988); and Ipsen, et al., *Allergy*, *43* : 370-7 (1988).

Patients allergic to birch pollen frequently (in up to 70% of the cases) also demonstrate an intolerance to fruits and/or vegetables. This syndrome has collectively been called an allergy to BPRF (birch pollen related food stuff)(Eriksson, et al., *Allergy*, *37* : 437-43 (1982). True cross-reactivity of IgE antibodies from apple-sensitive patients with Bet v 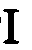 on western blot analysis, and binding of BIP I, a mouse monoclonal antibody specific for Bet v 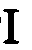 , to a 17 kD antigen from apple extract have been demonstrated (Ebner, et al., *J Allergy Clin Immunol*, *88* : 588-94 (1991).

In the case of the major airborne allergy caused by house dust, the offending allergens have been identified as proteins from three *Dermatophagoides species* : *Dermatophagoides pteronyssinus*, *Dermatophagoides farinae*, and *Dermatophagoides microceras*. The most important allergens from these *species* are referred to as Group 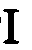 (respectively Der p 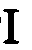 , Der f 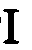 , and Der m 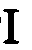 ) and Group 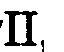 ( Der p 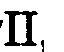 , Der f 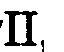 , and Der m 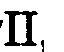 ). Complementary DNAs coding for those proteins have been cloned, and recombinant allergens have been expressed in various systems (for a review, see Baldo, B. A., *Curr Opin Immunol*, *3* : 841-50 (1991). It has been shown that human IgE antibodies react with both the Group 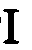 determinants that are common for the three mite species and the species-specific determinants (Chua, et al., *J Exp Med*, *167* : 175-82 (1988).

At present, treatment of those allergies is mainly directed to alleviation of symptoms, based on the usage of antihistamines and/or steroids. Classical immunotherapy, e.g., desensitization, despite its limited efficacy and its potential toxicity, will probably continue to be used. However, an efficient and effective treatment of the underlying allergic mechanisms would be highly desirable.

Specific modulation of the immune response to allergens could take advantage of the use of allergen-specific monoclonal antibodies. Classically, this would be done with non-human monoclonal antibodies (MAbs) such as murine antibodies. In fact, rodent MAbs to some of the allergens discussed above have been prepared, e.g., mAbs against Bet v 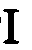 (Jarolim, et al., *Int Arch Allergy Appl Immunol*, *90* : 54-60 (1989). Such non-human MAbs may, however, elicit an antigenic response if used to treat allergic reactions in man.

It would therefore be more desirable to have human monoclonal antibodies (HuMAbs) which bind to these allergens and/or block the allergen-induced degranulation of IgE-sensitized basophils/mast cells. Single cross-reacting HuMAbs could even be used to treat allergy to distinct allergens which share common epitopes, as described above for tree pollens.

In the past, producing clones of immortalized human B-cells that secrete HuMAbs of a predetermined specificity has been difficult. To be able to obtain even one such clone after substantial effort may be considered unusual. It would thus be desirable to provide a process to produce a multiplicity of antibody-secreting human B-cell clones of a given specificity, from which multiplicity of cells a selection can be made to obtain one antibody or a set of HuMAbs with the desired characteristics. These clones and cDNA encoding the human monoclonal antibodies produced by such clones could be used to produce a wide variety of HuMAbs to specific antigens.

### SUMMARY OF THE INVENTION

The present invention fills the above-mentioned needs by providing methods and materials for the treatment of allergies. In particular, a first aspect of the present invention involves an anti-allergic agent comprising human monoclonal antibody (HuMAb) which binds to an airborne allergen that provokes an allergic IgE response, an allergen-binding fragment of such HuMAb, or an immune complex of such HuMAb or allergen-binding fragment with said allergen. The anti-allergic agent is a HuMAb of any human isotype (i.e., IgG, IgA, IgD, IgE, or IgM). However, it is preferably an IgG, IgA or IgD antibody; and more preferably, it is of the IgG class. The anti-allergic agent of the invention may be employed in pharmaceutical compositions in combination with a pharmaceutically acceptable carrier and in methods of treating allergic reactions.

In one embodiment, the anti-allergic agent is a HuMAb which binds to birch or house dust mite antigen. Preferably, it is a HuMAb which binds to the Bet v 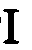 or Bet v 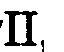 antigens of birch pollen or to the Der p 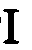 or Der p 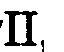 antigens of house dust mites.

In another embodiment, the anti-allergic agent comprises a HuMAb which inhibits the allergen-induced degranulation of IgE-sensitized basophils/mast cells, an allergen-binding fragment of such HuMAb, or an immune complex of such HuMAb or allergen-binding fragment with said allergen.

The invention also provides a human B-cell line established by CD40 cross linking and Epstein-Barr virus (EBV) transformation, which established cell line produces a HuMAb which binds to an airborne allergen that provokes an allergic IgE response and/or which inhibits the allergen-induced degranulation of IgE-sensitized basophils/mast cells. Preferably, a HuMAb-producing clone is isolated.

The invention further provides purified and isolated DNA which encodes the heavy and/or light chains of a HuMAb or allergen-binding fragment according to the invention.

A second major aspect of the invention is not limited to airborne allergens and more broadly relates to making HuMAbs to any desired antigen. In particular, a process is provided for making a HuMAb to a desired antigen comprising the steps of
establishing an immortalized human B-cell population from a patient having antibodies that bind to the desired antigen, said immortalization comprising infecting the B-cells with Epstein-Barr virus and crosslinking the CD40 of such B-cells;
culturing said immortalized B-cells;
isolating multiple clones from such immortalized B-cells, each of which clones secretes a human monoclonal antibody that binds to the desired antigen; and
using one or more of such clones to produce one or more human monoclonal antibody or an antigen-binding fragment of such human monoclonal antibody.
Preferably, nucleic acids derived from the clone which encodes said HuMAb or an antigen-binding fragment thereof are used to produce the desired HuMAb or antigen-binding fragment. The clone may also be hybridized with a (hetero)myeloma ,i.e., either a human myeloma or a hybrid human/mouse heteromyeloma), cell to produce a hybridoma that proliferates in culture and produces the desired HuMAb.

### DETAILED DESCRIPTION OF THE INVENTION

The invention may employ a B-cell population including resting B-cells which retain their surface bound immunoglobulin and/or activated B-cells which secrete HuMAbs. If desired, the B-cell population may be sorted to select for activated B-cells or for resting B-cells, e.g., as described below and in WO 91/09115.

A starting human B-cell population for use in accordance with the present invention, e.g., in providing an anti-allergic agent, can be identified using means conventional in the art. A small amount of blood can be taken from patients and tested for Ig against the desired antigen, e.g., an airborne allergen. Patients who react positively in a conventional skin test and/or a RAST® or a CAPS® (available from Pharmacia, Sweden) with an antigen, e.g., an airborne allergen of interest, are sources of B-cells that can be used to immortalize and isolate a clone producing the desired HuMAb as described further below. Alternatively, B-cells from a patient who tests negatively in such tests, but nevertheless has serum that tests positive for an antibody to the antigen, e.g., by ELISA, radioimmunoprecipitation assay, western blotting, etc., may also be employed as a starting B-cell population. A larger sample can then be taken from each patient identified by the above procedures.

Suitable sources of B-cells from a selected patient include peripheral blood, tonsils, adenoid tissue, spleen (in the case of removal for another medical necessity) or any other source of B-cells from the body. Typically, peripheral blood is employed as the B-cell source.

The blood is first treated to separate the peripheral blood lymphocytes (PBLs) from the red blood cells and platelets by means conventional in the art. For example, the peripheral blood may be diluted with an appropriate isotonic medium, e.g., RPMI 1640 medium (cat. 041-01870 M. Gibco, USA). The diluted blood is loaded onto a suitable separation medium such as FICOLL® (available from Pharmacia, Sweden). After centrifugation, the PBLs may be aspirated from the interface between the serum and the FICOLL®. The purified PBLs may be frozen in liquid nitrogen for later use. The plasma is then analyzed by conventional techniques such as ELISA, radioimmunoprecipitation assay, western blotting, etc., to confirm the presence of significant amounts of the desired antibody (e.g., IgG and/or IgE antibody) against the antigen of interest, e.g., an airborne allergen such as the 17 kD allergen of the pollen extract Bet v 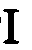 .

The purified PBLs may be used directly or may be further enriched and/or sorted as discussed below. For example, T-cells may be removed by rosetting with 2-aminoeylthisothiouronium bromide-treated sheep erythrocytes. Further selection for an antigen-specific B-cell subpopulation can be carried out by a variety of techniques including panning, immunoadsorbent affinity chromatography, fluorescent-activated cell sorting (FACS), etc. These techniques are described for example in Casali et al., *Science*, *234* : 476-479 (1986); U.S. patent No. 4,325,706; and Mage, Hubbard et al., Parks et al. and Haegert, in *Meth*. *Enzymol*., *108* : 118-124, 139-147, 197-241 and 386-392 (1984), respectively. The PBLs may also be treated with magnetic beads whose surface is coated with a material to selectively sort the desired B-cells. Such beads may be coated, e.g., with anti-Ig isotype for the desired Ig to be separated, anti-surface antigen to select for non-naive B-cells, or a purified antigen or allergen.

The resulting enriched and/or sorted B-cell population is then subjected to the B-cell immortalization process described in WO 91/09115. Briefly, the B-cells are transformed with Epstein-Barr virus (EBV) and their CD40 molecules are crosslinked. Reference is made to WO 91/09115 for the variations that may be employed in this activation/immortalization process.

The treated B-cell population may be washed by an appropriate isotonic medium( e.g., with RPMI 1640), pelleted and then resuspended in medium. The cells are then transformed with EBV by the addition of a suitable EBV strain, preferably a strain such as the one released by the B95.8 cell line available from the ATCC (ATCC CRL 1612). The amount of EBV used may vary depending on the strain of the virus and the number of B-cells to be transformed. For example, with a sample containing 14x10⁶ non-sorted PBLs, 200 µl of a concentrated EBV (strain B95.8) suspension is typically used. Incubation with the virus is typically carried out for about 1 to 24 hours, preferably for about 2 hours, at 37°C; but other conditions may be employed, if desired.

The EBV-infected cells are preferably washed and resuspended in an appropriate medium such as LINOLEA® 15% Fetal Calf Serum (FCS) [Yssel, et al., *J*. *Immunol*. *Methods*, *72* :219-24 (1984)] or DMEM-F12 (cat. 041-01331M, Life Technologies, Paisley, Scotland) 15% Horse Serum. The concentration of the PBLs in the suspension may vary depending, for example, on whether a sorting step for antigen-specific B-cells was performed as described above. Lower concentrations can be employed when PBLs have been enriched in the desired B-cells. Typical concentrations for non-selected PBLs are from about 1x10³ to about 5x10⁴ cells/ml. If the B-cell population is first sorted as discussed above, the concentration may be decreased depending upon the efficiency of the sorting, e.g., up to about 1x10² cells/ml.

An agent capable of crosslinking CD40 antigen is added to the suspended cells. The crosslinking agent may include T-cells, other transfected cells expressing CD40 ligand or membranes therefrom. Other suitable agents are described in WO 91/09115. Preferably, the agent is an immobilized monoclonal antibody specific for the CD40 antigen, e.g., immobilized on irradiated fibroblasts expressing the human or murine Fc-gamma receptor.

The monoclonal antibody to CD40 can be any which binds to the CD40 marker on the B-cells of the suspension and also to the Fc-gamma receptor of the L-cells. Preferably, the monoclonal antibody is selected from MAb 89 and G28-5. These antibodies are described in Valle et al., *Eur*. *J*. *Immunol*, *19* : 1463-1467 (1989) and Ledbetter et al., *J*. *Immunol*, *138* : 788-794 (1987), respectively. The hybridoma corresponding to MAb 89 has been deposited with the European Collection of Animal Cell Cultures, PHLS Centre for Applied Microbiology and Research, Porton Down, Salisbury Wilts. SP4 OJG, U.K. under accession No. 89091401.

Typically, the PBLs, the CD40 antibody and the L-cells are simply mixed together in appropriate amounts. The CD40 antibody may be present in a concentration of from about 0.1 µg/ml to about 5 µg/ml, preferably about 0.5 µg/ml.

The treated cell suspension is divided among an appropriate number of wells of a tissue microplate to provide a suitable cell concentration for amplification and screening. If enriched suspensions are employed as the result of an antigen-selective screening as discussed above, fewer cells per well may be used.

Typically, the initial culture phase takes 10-20 days in the case of non-selected PBLs and 5 days or even less in the case of an antigen-specific enriched B-cell population, which would allow an earlier detection of specific antibodies. During this phase, fresh medium is added as necessary. The duration of this initial culture phase is adjusted to allow detection of the antigen-specific B-cells, while preventing them from being overgrown by non-specific B-cells. A sample of supernatant from each well is screened by an appropriate assay for the desired HuMAb positive characteristics, e.g., by ELISA, western blotting, radioimmunoprecipitation assay, etc.

Cell lines which test positive for the desired HuMAb characteristics are subcloned (3-10 cells/well) and cloned (0.5-1.0 cells/well) by techniques well-known in the art, e.g., by culturing in limiting dilution conditions for 7-20 days in additional medium as needed. Supernatants of the clones are screened by the procedures described above.

Positive clones are expanded in a larger volume and amplified by conventional incubation. When no positive clones are found, the subclones are screened and the positive ones are used for additional cloning.

HuMAb can be purified from the supernatant of the amplified clones by conventional immunoglobulin purification methodology. For example, the HuMAb may be precipitated with solid ammonium sulfate, reconstituted in sterile water, and dialyzed extensively against a buffer such as phosphate buffer saline (PBS). The dialysate may then be applied to an immunoaffinity column, e.g., a column having anti-human Ig covalently coupled to Sepharose. After washing, the desired HuMAb may be eluted from the column by any appropriate eluent, e.g., acidic buffer, chaotrophic agents, etc. [for example, see in Current Protocols in Immunology, edited by John E. Coligan et al., John Wiley and Sons, New York]

By the term "human monoclonal antibody" as used herein, we mean to include HuMAbs that are isolated from human B-cells as discussed above (e.g., whether the antibody is prepared by culturing the immortalized human B-cells or recombinantly from human B-cell cDNAs encoding such a HuMAb) and HuMAbs that are made by recombining the variable portions of a HuMAb of the present invention of one isotype (e.g., an IgE) with the constant region of a human antibody of another isotype (e.g., a human IgG). Recombinant methods for making these HuMAbs are described below.

By the terms "antigen-binding fragment" or "allergen-binding fragment", we mean an antibody fragment such as an Fab, (Fab)₂, Fv, single chain binding protein, or any other binding polypeptide which contains one or more complementarity determining regions (CDRs) of the variable region of a light or heavy chain of a HuMAb of the present invention. These fragments can be prepared by well-known methods. For example, fragments can be made from the full length HuMAb protein, e.g., by papain or pepsin cleavage, or by chemical oxidation, followed by separation of the resulting fragments. Alternatively, recombinant DNA technology may be used. For example, cDNA encoding the variable regions of both heavy and light chains may be engineered to produce the Fv portion of the HuMAb of the invention. See, for example, the methodology of U.S. patent No. 4,642,334 which may be employed.

By the term "immune complex" as used herein, we mean a complex of an airborne allergen and its corresponding HuMAb or allergen-binding fragment in accordance with the present invention. These immune complexes may be prepared by conventional techniques. For example, a predetermined weight ratio such as a one to five molar ratio of purified or recombinant allergen (or a fragment thereof) and of HuMAb(s) binding to it may be incubated for 2 hours at 37°C, and stored for later use at -80°C.

The B-cell clones of the invention may be used in conventional DNA recombinant methods to produce the HuMAbs of the invention or fragments thereof. For example, RNA from the B-cell clones may be isolated according to the single-step method described by (Chomczynski, et al., *Anal Biochem*, *162* : 156-9 (1987). Briefly, about 10⁷ cells are lysed in guanidinium thiocyanate denaturing solution. After acidification of the mixture with 2M sodium acetate, pH4, RNA is extracted with phenol and chloroform/isoamyl alcohol. RNA is then precipitated with isopropanol, the RNA pellet is redissolved in denaturing solution, reprecipitated with isopropanol, and washed with 75% ethanol.

cDNA is obtained by reverse transcription, e.g., using the Superscript Reverse Transcriptase Kit (cat. 20898 BRL, Gaithersburg, MD, USA), with random hexamer primers. The cDNA is then used as template in the polymerase chain reaction (PCR). The primers may be designed to include restriction sites, to allow for the directional cloning of the PCR products. For the heavy chain, primers specific for the leader sequence of the 6 different human VH families are used individually in conjunction with primers located at the 3' end of the constant region corresponding to the isotype previously determined by isotyping the HuMAb by ELISA. The light chain is amplified with individual combinations of primers corresponding to the 3' end of the kappa or lambda chain in conjunction with a series of primers annealing to the leader sequence of the V kappa or the V lambda genes. Thus, full length heavy and light chains starting at the initiation codon in the leader sequence and ending at the stop codon may be generated.

After appropriate restriction cleavage, both full length heavy and light chains can then be cloned in any appropriate expression vector designed to be compatible with the restricted PCR products. Appropriate vectors include for example baculovirus vectors and plasmids compatible with CHO cells. Heavy and light chains can be cloned individually in distinct vectors, or in tandem in one vector. The recombinant plasmids or viral vectors may be cloned in bacteria, and a few clones may be sequenced on both strands to check for the absence of alteration of the insert. One clone each for the heavy and the light chain, or one clone containing both chains may then be selected for expression in the appropriate host cells. Depending on the vector used, it will be introduced in appropriate prokaryotic or eukaryotic cells either by transfection or infection. After cloning the cells expressing the recombinant HuMAb, supernatant fluid from the cultured cells is collected, and the HuMAb therein can be purified, e.g. by immunoaffinity, HPLC or any other appropriate methods.

The full length PCR product for the heavy chain can be modified for example to replace the original heavy chain constant region by another one, so substituting, e.g., a IgE isotype to IgG isotype. This can be accomplished through a second PCR using a primer annealing to the 3' end of the VH region (e.g., IgE) and including a tail corresponding to the 5' end of the desired constant region (e.g., IgG) cloned in an appropriate plasmid. After amplification, the heavy chain coding DNA generated may be digested with appropriate enzymes and ligated into the new expression vector (containing the sequence of the desired heavy chain, e.g., IgG). This will allow the production of a recombinant HuMAb with the variable region of the originally isolated HuMAb (e.g., the variable region from an IgE HuMAb) and the constant region of a different human isotype (e.g., the constant region of a human IgG). This type of recombined HuMAb will have the characteristic binding of the IgE HuMAb, but will be able to display the effector functions normally associated with the human IgG isotype.

Hybridomas may also be made with the B-cells of the invention by techniques conventional in the art. For example, the B-cells of the invention may be fused with an appropriate myeloma cell or with a heterohybridoma cell to increase or stablize the immunoglobulin secretion. See for example Kudo et al., *J*. *Immunol*. *Methods*, *145* : 119-125 (1991); and Zanella et al., *J*. *Immunol*. *Methods*, *156* : 205-215 (1992).

The anti-allergic agent of the present invention may be used therapeutically to treat existing symptoms associated with the antigen of interest, e.g., an allergic reaction, or prophylactically to prevent or inhibit the occurrence of such symptoms. The anti-allergic agent may be used alone or in combination with at least one other anti-allergic agent to form a cocktail. For example, such a cocktail may include two or more of the HuMAbs of the invention, each of which binds to one or more epitopes on an antigen of interest. When such a cocktail is employed, the proportions of the various HuMAbs may vary depending, for example, upon their binding characteristics and/or ability to inhibit mast cell/basophil degranulation.

The HuMAbs or fragments of the invention may be administered as an immune complex with the sensitizing antigen, e.g., a complex of an anti-Bet v 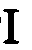 HuMAb with the Bet v 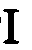 antigen. These immune complexes can be prepared in accordance with the procedures described in (Jacquemin, et al., *Lancet*, *335* : 1468-9 (1990); and Machiels, et al., *J Clin Invest*, *85* : 1024-35 (1990); and EP 287361).

The HuMAbs, fragments and/or immune complexes are preferably administered in the form of a pharmaceutical composition containing a therapeutically or prophylactically effective amount of at least one such HuMAb, fragment or immune complex in combination with a pharmaceutically acceptable carrier. Any appropriate carrier may be employed, i.e., a compatible, non-toxic material suitable for delivery of the HuMAb or immune complex in the desired dosage form, e.g., oral, parenteral (subcutaneous, intramuscular or intravenous), or topical dosage forms. Suitable carriers include sterile water, sterile buffered water, sterile saline, etc. Special pharmaceutical compositions to insure a sustained release of the HuMAb, fragment and/or of the immune-complex may also be employed.

The concentration of the HuMAb, fragment or immune complex of the invention in the pharmaceutical compositions may vary, e.g., from about 0.1 µg/ml to about 1 mg/ml, preferably from about 1µg/ml to about 100 µg/ml. The concentration used will depend upon the number of HuMAbs, fragments and/or immune complexes employed in the composition and the dosage form selected, and the dose will be adjusted in a conventional manner by the skilled artisan to levels appropriate to achieve the desired result *in vivo*.

As mentioned above, the anti-allergic agent of the invention can be used therapeutically or prophylactically. Thus, the agent may be administered before the onset of symptoms of the allergic reaction or after the symptoms have appeared. When used prophylactically for seasonal allergy, the anti-allergic agent of the invention may be administered before the start of the allergic season for the particular allergen of interest, preferably from about 4 months to about 2 weeks before the expected start of the allergen's season. When used therapeutically, the anti-allergic agent is administered on an appropriate schedule to relieve the allergic symptoms. Typically the anti-allergic agent should be administrated so as to protect the patient continuously during the allergenic season. This may be achieved either by daily topical (e.g., intranasal) administration, by parenteral injection every 2 to 3 weeks, or by few injections of a slow releasing formula mentioned above. The anti-allergic agent of the invention will be administered in a dose effective to alleviate the allergic effect. Amounts effective for this purpose will depend upon many factors, e.g., the sensitivity of the patient or the load of allergen to which the patient has been or will be exposed. In case of allergy to perennial allergens such as house dust mites, a primary prevention could be attempted by the administration of the anti-allergic agent before the onset of the symptoms in a population known to be at risk for the development of florid hypersensitivity, i.e., children with high serum IgE levels and allergic parents.

The anti-allergic agent or a HuMAb or fragment thereof produced in accordance with the processes of the invention may be administered in dosages of from about 0.001 µg/kg to about 1 µg/kg, preferably, from about 0.01 µg/kg to about 0.1 µg/kg. The proper dosage of an agent of the invention for a particular situation will be determined by using common practices in the art. Generally, treatment may be initiated with smaller dosages that are less than the optimum dose of the agent. Thereafter, the dosage may be increased by small increments until the optimum effect under the circumstances is reached. The amount and frequency of administration of the agents of the invention will be regulated according to the judgment of the attending clinician considering such factors as age, condition and size of the patient as well as severity of the symptom being treated.

The HuMAbs and fragments of the invention may also be used for diagnostic purposes in the same manner as other antibodies and fragments thereof are currently used in the art. For example, the HuMAbs and fragments of the invention can be used in assays for the antigen, e.g., airborne allergens, to which they bind. The HuMAbs and fragments may be used labeled (e.g., with a radioisotope, fluorescent group, enzyme or other appropriate ligand) or unlabeled as is conventional in the art for the particular assay of interest (e.g., in a sandwich assay with a second labeled antibody). The HuMAbs and fragments may be used in agglutination assays, enzyme immunoassays, etc. They could for example be used to calibrate a dosage of antigen-specific IgG in the serum or in any other biological fluid. Thus, the labeled or unlabeled forms of the HuMAbs and fragments of the invention may be employed as elements of kits for purposes of performing the desired assay.

The invention disclosed herein is exemplified by the following examples, which should not be construed to limit the scope of the disclosure. Alternative methods within the scope of the invention will be apparent to those skilled in the art.

### EXAMPLES

Unless otherwise indicated, percentages for solids in solid mixtures, liquids in liquids, and solids in liquids are expressed on a weight/weight, volume/volume and weight/volume basis, respectively.

### GENERATION OF HUMAN CLONES SECRETING ANTIBODIES WHICH SELECTIVELY BIND TO BIRCH POLLEN ALLERGENS

Clinical selection of adult patients was first based on allergic symptoms (rhinitis, conjunctivitis, asthma) occurring early in the spring. Involvement of birch pollen in causing the symptoms was then confirmed by the PRIST® measurement of the total IgE in their sera, and by the presence of birch-pollen specific IgE antibodies as demonstrated by skin test reactivity and CAPS® (PHARMACIA, SWEDEN) measurement. Ten allergic patients were selected, according to these criteria.

Fifty ml of blood from each selected patient was collected in a tube containing EDTA as an anticoagulant, then diluted 1:1 with RPMI medium. This diluted blood was loaded onto FICOLL® (Pharmacia, Sweden) according to manufacturer's instruction, and after centrifugation, the PBLs were harvested at the interface. The upper phase was recovered, aliquoted, and then frozen at -20°C. This diluted (1:1) plasma was used later for further analysis by ELISA and western blotting. Purified PBLs were frozen at a concentration of 1x10⁶ PBLs/ml and kept in liquid nitrogen. The diluted sera were analyzed by ELISA and Western blot (see below). The PBLs from one patient were selected for initial transformation on the basis of the presence of large amount of IgG against birch pollen proteins, and the presence of both IgG and IgE antibodies against the 17 Kd allergen of the pollen extract (Bet v 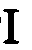 ).

14x10⁶ PBLs from the selected patient were thawed and washed once in RPMI. Viability, as estimated by Blue Trypan exclusion, was above 90%. The cells were pelleted, resuspended in 1 ml of RPMI 1640. Two hundred µl of concentrated EBV suspension (strain B95.8) were added, and this mixture was incubated for 2 hours at 37°C. The cells were washed twice in RPMI 1640, and then resuspended at 5 x10⁴ PBLs/ml in LINOLEA®-15% Fetal Calf Serum (FCS). Irradiated L-cells (3000 rads) expressing the human Fc-gamma receptor were added at a final concentration of 5 x10⁴ cells/ml, together with the murine monoclonal anti-human CD40 antibody mAb 89 at a final concentration of 0.5 µg/ml. One hundred µl aliquots of this mixture were then distributed in each well of Microwells 96U round-bottom plates (Nunc ref. 1-63320, Denmark), and the plates were incubated at 37°C in a 5% CO₂ atmosphere. After 5 days of incubation, 75 µl of fresh medium were added to each well. At day 12, the first screening of supernatant was performed by collecting 100 µl of medium from each well and individually performing an ELISA (see below) for the presence of IgG antibodies binding the birch pollen proteins. One hundred µl aliquots of fresh medium were added to the wells. At day 19, the same screening was repeated on every well to confirm the first assay, and to identify lines which had subsequently become positive.

A total of 51 positive lines were thus identified, all of which were transferred in 2 ml of fresh LINOLEA®-15% FCS to 24 well plates for amplification. After expansion of the lines, 5 x10⁶ cells from each were frozen and kept in liquid nitrogen. Twenty-five lines were kept in culture for further expansion, characterization of antigenic specificity, and cloning. Two lines were found by western blotting to secrete antibody binding to birch pollen proteins of molecular weight 17 Kd and 14 Kd, respectively. These lines were cloned by limiting dilution in Microtest III Tissue Culture Plates (Falcon, New Jersey, USA) flat-bottom microwells. After subcloning and cloning, one clone was derived from each of those two lines. One clone - designated G5F2A4 - secreted an antibody binding to Bet v 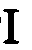 . The other clone - designated G25E8B5 - produced an antibody binding to Bet v 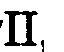 . Both clones were maintained in culture for more than 4 months and showed stable proliferation. Periodic analysis of antibody secretion during and after cloning confirmed that the lines and clones had conserved their secretory capacity and their specificity.

Analysis of the supernatant fluids of the clones for IgG subclass isotyping showed that G5F2A4 secreted an IgG1 antibody, while G25E8B5 secreted an IgG4 antibody. PCR amplification of cDNA derived from G25E8B5 with primers specific for the different VH families established that the heavy chain secreted by this clone has a rearranged VH3 region. Likewise, PCR amplification with primers specific for the light chains established that only kappa chain is productively rearranged.

### ASSAYS

All the work with the birch pollen was carried out using natural antigen, to ensure the clinical relevance of the allergens studied (i.e., to ensure that the allergens had native conformation and glycosylation). We used a pollen extract commercialized for the bronchial challenge of patients (BIRCH POLLEN INHALATION, Smith Kline Beecham Pharmaceuticals, London, U.K.)

### Characterization of the pollen extract:

The total protein concentration of the birch pollen extract was estimated by the absorption at 280 nm of the solution, and this estimation was then confirmed using the Bio-Rad DC Protein Assay kit (Bio-Rad, Rihmond, CA). The concentration of protein was found to be 100 µg/ml. To make an estimation of the proportion of Bet v 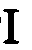 in the extract, we ran a 12% acrylamide SDS-PAGE gel electrophoresis under reducing conditions. Several dilutions of the pollen extract were loaded on a gel, as well as a known amounts of low molecular weight protein standards. After completion of the run, the gel was silver stained, and a visual estimation of the concentration of Bet v 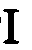 was made by the comparison with the molecular weight standards. It was estimated that the Bet v 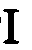 concentration was about 5 µg/ml in the pollen extract, and that it represented about 10 to 20 % of the proteins in the extract.

### Indirect ELISA for the detection of anti-birch pollen HuMAbs

After testing several dilutions of the birch pollen extract, the extract was diluted 1:200 in coating buffer (Carbonate Buffer, pH:9.6, Na₂CO₃, 15mM, NaHCO₃, 35mM). This diluted solution was coated onto the wells of 96 microwell ELISA plates (Immunoplate Maxisorp F96 certified, NUNC, Denmark) overnight at room temperature. The plates were then washed manually once with washing buffer: Phosphate Buffer Saline, Tween 20 (cat T21-0309 Technicon Diagnostics, USA) 0.05%. Sera were diluted 1 to 100 in dilution buffer, TBS-B-T buffer: Tris 20mM, NaCl 150 mM, Bovine Serum Albumin (BSA, cat A-9647 Sigma St Louis MI, USA) 1%, Tween 20 0.05%, and 100 µl of diluted sera were added to each of the wells. Supernatant fluids from the lines and clones were used undiluted. Sera or supernatants were incubated for 2 hours at 37°C, then washed once as described above. 100 µl aliquots of rabbit anti-human IgG coupled to alkaline phosphatase (cat D336 Dakopatts, Denmark) were added to each well at a 1:2000 dilution in TBS-B-T buffer. The antibodies were incubated for 1 hour at 37°C. Wells were then washed three times with washing buffer. 100 µl of alkaline phosphatase substrate (p-nitrophenyl-phosphate, disodium, 1mg /ml of 10% diethanolamine solution, pH 9.8) was then added to each well, and the colorimetric reaction was read at 405/490 nm. Background was determined as the optical density (O.D.) of control wells without human antibodies.

An Indirect ELISA for the detection of specific IgE antibodies in the sera was carried out in the same way, except that incubation of the sera diluted 1 to 30 in dilution buffer was for 4 hours at room temperature. For the detection, a mouse monoclonal anti-human IgE antibody prepared by standard techniques and designated IgE 127 was diluted 1 to 5000 in TBS-B-T buffer, and 100 µl were added and then allowed to incubate for 4 hours at room temperature. After one wash, 100 µl of peroxidase-labeled goat anti-mouse Ig (cat 6540 TAGO, Burlingame, CA, USA) diluted 1 to 3000 in TBS-B-T buffer were added and the mixture was incubated for 1 hour at 37°C. The wells were washed three times, after which 100 µl of the peroxidase substrate were added. After 15 to 30 minutes, absorbance was measured at 405 nm.

### Western Blot assay

15 % SDS-acrylamide gels (14 x 12 x 0.75 cm) were used with O'Farrell/Laemmli Tris-Glycine-SDS buffer (BufferEZE formula 2, cat 8327603 Kodak, Rochester, NY, USA). Natural birch-pollen proteins were treated with 2-mercaptoethanol, loaded at 2 µg/cm, and subjected to electrophoresis.

Proteins separated by SDS-PAGE were transferred to 0.45 micron nitrocellulose membrane (Hybond-C super, Amersham, UK) using a Tris 25mM, glycine 192mM, Methanol 20% buffer. Transfer was performed with a BIOLYON cell (cat 50111, Biolyon, Dardilly, France) for 40 minutes at 250 mA. After transfer, unbound membrane sites were blocked by immersion with rocking in Tris 10mM , NaCl 500mM, Tween 20 0.05%, BSA 3%, pH 7.4, for 1 hour at room temperature.

Nitrocellulose strips (2 mm in width) were incubated with atopic sera (diluted 1:20 in Tris 10mM, NaCl 500mM, Tween 20 0.05%, pH 7.4 , BSA 1%) or with undiluted culture supernatants overnight at 4 °C. After washing twice with Tris 10mM , NaCl 500mM, Tween 20 0.05% (TNT) for 30 minutes, one of the following procedures was used for detection:
(1) *chemiluminescence* : For the detection of IgE, the strips were incubated with the murine monoclonal anti-human IgE 127 (dilution 1:5000 in TNT) for 1 hour at room temperature with rocking. After washing, goat anti-mouse Ig peroxidase conjugate (TAGO ref. 6540 diluted 1 :500 in TNT) was added for 1 hour at room temperature. After washing, detection was performed with the ECL Western blotting detection reagents (cat 2106 Amersham, UK) according to the manufacturer's instructions. The strips were then loaded into a Kodak X O-matic cassette together with a photographic film (X O-mat AR, KodaK).
(2) *peroxidase* : Nitrocellulose strips were incubated for 1 hour at room temperature with peroxidase-labeled sheep Fab anti-human IgG (cat 1087-690 Boehringer Mannheim GmBH, W.-Germany) diluted 1:2000 Tris 10mM , NaCl 500mM, Tween 20 0.05%, pH 7.4 , BSA 1%. After washing as above, color development was performed with 3,3'-diaminobenzidine tetrahydrochloride(cat D-5905 Sigma, St. Louis MI USA) 0.1%, NiCl₂ 0.003%, H₂O₂ 0.03%.

### Immunoprecipitation of purified Bet vI

Bet v 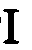 is purified by immunoaffinity column: a murine mAb against Bet v 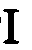 is prepared by conventional techniques and covalently coupled to an AFFIGEL HZ® hydrazide gel column (cat 153-6048, Biorad, Richmond, CA. USA). Purified Bet v 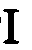 is then iodinated with the Iodo Beads kit (cat 28665X, Pierce, Rockford, USA). After iodination, the labeled material is subjected to SDS-PAGE in a 15% gel, which is dried after completion of the run. An X O-Mat film is exposed to the dried gel, to check for the purity of the iodinated agent, and to control the efficacy of the labeling.

Radiolabeled Bet v 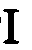 (20.000 CPM) is incubated for 1 hour at 37°C with diluted sera or with the supernatants from lines or clones. This mixture of antigens and antibodies is incubated for 1 hour at 37°C in the presence either of protein A or anti-isotype antibody coupled to beads. After incubation, the beads are washed three times, to remove unbound radioactivity. After washing, the bound radioactivity is measured in a gamma counter. Specificity of the precipitation of the radioactivity is confirmed through its inhibition by the addition of high concentrations of unlabeled Bet v 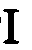 at the same time as the iodinated allergen. This cold Bet v 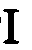 inhibits the precipitation of radiolabeled Bet v 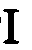 .

### Specific IgE inhibition (CAPS inhibition)

CAPS® (Pharmacia, Sweden) bearing the birch pollen are incubated at 37°C for 2 hours with different dilutions of the monoclonal antibody in the buffer provided by the manufacturer. The CAPS® are then used with the sera of patients which have previously been shown to contain IgE antibodies against Bet v 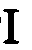 only, as judged from a western blot assay as described above. The CAPS® are further processed according to the manufacturer's instructions with an automatic CAPS® machine (Pharmacia, Sweden). Specific IgE inhibition is measured by comparing the radioactivity bound to the CAPS with and without pre-incubation with the HuMAbs.

### Inhibition of basophil degranulation

Histamine release analysis is performed on whole blood from patients allergic to the birch pollen. 10 ml aliquots of blood are collected into tubes containing heparin. The histamine is measured with an Enzyme Immunoassay Kit (cat 1153, Immunotech France). The dosage of total histamine after cell lysis, and the spontaneous release of histamine are performed according to the manufacturer's instructions.

Cells are challenged with the allergen by diluting the whole blood 1:7 in histamine release buffer. 50 µl of different dilutions of the pollen extract or of the purified allergens are added to the wells of the microtiter plate. 100 µl of the 1:7 dilution of whole blood is added to each well, and this is incubated for 30 minutes at 37°C. Inhibition measurements are made in the same way, except that the different dilutions of the allergens are first incubated for 1 hour at 37°C with the HuMAb at a final concentration of 1 mg/ml. The "protected" allergens are then added to the wells of the microtiter plates as described above. Every dosage is done in duplicate. Results are obtained by extrapolation from a standard curve.

### Skin test inhibition in monkeys

Passive sensitization of the skin of monkeys is performed by intradermal injection of 1 ml of the serum from allergic patient in one forearm. 24 hours later, 10 ml of Blue Evans dye is administered to the animal just before challenge by prick skin test with serial dilutions of the birch pollen (Bet v 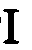 ) extract in the area of pre-sensitized skin. The specific reactions are evaluated by comparison of the size of the flare and weal reaction elicited with the same series of skin prick tests in the control non-sensitized forearm.

Inhibition of passive cutaneous anaphylaxis is determined by the effect of injection of the HuMAb secreted by clone G5F2A4 at different times and dosages. The HuMAb is administrated either locally, in the sensitized area, just before the skin test, or systematically, e.g. by iv or im injection. The HuMAb is also administrated as a mixture at different ratios with the challenging allergen in a series of skin tests.

While the present invention has been described in conjunction with the specific embodiments set forth above, many alternatives, modifications and variations thereof will be apparent to those of ordinary skill in the art. All such alternatives, modifications and variations are intended to fall within the spirit and scope of the present invention.

## Claims

1. An anti-allergic agent comprising human monoclonal antibody which binds to an airborne allergen that provokes an allergic IgE response, an allergen-binding fragment of such human monoclonal antibody, or an immune complex of such human monoclonal antibody or allergen-binding fragment with said allergen.

2. An anti-allergic agent according to claim 1 which is a human monoclonal antibody of the IgG class.

3. An anti-allergic agent according to claim 1 which is a human monoclonal antibody which binds to birch or house dust mite antigen.

4. A human monoclonal antibody according to claim 3, wherein the allergen to which the antibody binds comprises Bet v 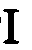 , Bet v 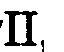 , Der p 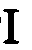 or Der p 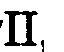 .

5. An anti-allergic agent comprising human monoclonal antibody which inhibits the allergen-induced degranulation of IgE-sensitized basophils/mast cells, an allergen-binding fragment of such human monoclonal antibody, or an immune complex of such human monoclonal antibody or allergen-binding fragment with said allergen.

6. A human B-cell line established by EBV transformation and CD40 cross linking, which established cell line produces a human monoclonal antibody according to any one of claims 1-5.

7. A human monoclonal antibody-producing clone of an established human B-cell line according to claim 6.

8. Purified and isolated DNA which encodes the heavy and/or light chains of a human monoclonal antibody or allergen-binding fragment according to any one of claims 1-5.

9. A pharmaceutical composition comprising at least one anti-allergic agent according to any one of claims 1-5 and a pharmaceutically acceptable carrier.

10. The use of an anti-allergic agent according to any one of claims 1-5 or a mixture of such agents for the preparation of a pharmaceutical composition to treat allergic reactions.

11. A process for making a human monoclonal antibody to a desired antigen comprising the steps of
establishing an immortalized human B-cell population from a patient having antibodies that bind to the desired antigen, said immortalization comprising infecting the B-cells with Epstein-Barr virus and crosslinking the CD40 of such B-cells;
culturing said immortalized B-cells;
isolating multiple clones from such immortalized B-cells, each of which clones secretes a human monoclonal antibody that binds to the desired antigen; and
using one or more of such clones to produce one or more human monoclonal antibody or an antigen-binding fragment of such human monoclonal antibody.

12. A process according to claim 12, wherein nucleic acid encoding a human monoclonal antibody or an antigen-binding fragment thereof is used to produce the desired human monoclonal antibody or antigen-binding fragment.

13. A process according to claim 12, wherein said clone is hybridized with a myeloma or heteromyeloma cell to produce a hybridoma that proliferates in culture and produces the desired human monoclonal antibody.

14. A human monoclonal antibody produced by a process according to any one of claims 12- 13
